**Europäisches Patentamt**

**European Patent Office**

**Office européen des brevets**

(19)

(11) Veröffentlichungsnummer: **0 306 745 B1**

# EUROPÄISCHE PATENTSCHRIFT

(45) Veröffentlichungstag der Patentschrift: **05.08.92**

(51) Int. Cl.⁵: **A61F 2/38**

(21) Anmeldenummer: **88113470.4**

(22) Anmeldetag: **19.08.88**

(54) **Tibiateil einer Kniegelenk-Endoprothese.**

(30) Priorität: **09.09.87 DE 3730174**

(43) Veröffentlichungstag der Anmeldung:
**15.03.89 Patentblatt 89/11**

(45) Bekanntmachung des Hinweises auf die
Patenterteilung:
**05.08.92 Patentblatt 92/32**

(84) Benannte Vertragsstaaten:
**AT BE CH DE ES FR GB GR IT LI LU NL SE**

(56) Entgegenhaltungen:
**DE-C- 3 433 264**

(73) Patentinhaber: **S + G IMPLANTS GMBH**
**Grapengiesserstrasse 34**
**W-2400 Lübeck(DE)**

(72) Erfinder: **Grundei, Hans**
**Hamburger Strasse 89**
**W-2400 Lübeck(DE)**
Erfinder: **Nicola de, Ugo, Dr.**
**Breslauer Strasse 35**
**W-Bad Mergentheim(DE)**

(74) Vertreter: **Eisenführ, Speiser & Partner**
**Martinistrasse 24**
**W-2800 Bremen 1(DE)**

Rank Xerox (UK) Business Services

## Beschreibung

Die Erfindung geht aus von einem Tibiateil einer Kniegelenk-Endoprothese, bei dem in die beiden vorn durch eine Brücke zusammenhängenden im Schienbein zu verankernden, metallischen Schalen die Tibiaplateaus mit Gleitflächen für die Kufen des Femur-Endoprothesenteiles eingesetzt sind. Tibiateile mit Schalen der vorerwähnten Art sind zum Beispiel durch die DE-C 34 33 264 bekannt.

Bei natürlichen Kniegelenken, die wegen Verschleißes oder dergleichen durch eine Kniegelenk-Endoprothese mittels operativen Eingriffs zu ersetzen sind, kann der Fall vorliegen, daß die beiden natürlichen Kreuzbänder so weit verschlissen sind, daß sie während der Operation nicht wieder fixiert werden können und dann durch künstliche Kreuzbänder ersetzt werden müssen, für die die Befestigung der Enden aber nicht möglich ist. Man hat dann auf Kniegelenk-Endoprothesen zurückgegriffen, die scharnierartig miteinander im Eingriff stehen.

Die Aufgabe der Erfindung besteht darin, solche scharnierartig miteinander im Eingriff stehenden Endoprothesen zu vermeiden und die Kniegelenk-Endoprothese so auszubilden, daß künstliche Kreuzbänder bei vereinfachtem operativen Eingriff einfach und leicht am Tibiateil der Kniegelenk-Endoprothese befestigt werden können.

Diese Aufgabe wird nach der Erfindung bei dem eingangs erwähnten Tibiateil dadurch gelöst, daß der Raum zwischen den beiden Schalen von einem künstlichen Höckersteg ausgefüllt ist, der hinten durch Formschluß mit den Schalen verbindbar ist und vorn durch eine Klemmverbindung an der Schalenbrücke befestigt ist und der mit den Enden von künstlichen Kreuzbändern verbindbar ist. Es wird also mit den beiden, die beiden Tibiaplateaus aufnehmenden Schalen ein künstlicher Höckersteg zur Befestigung der einen Enden der beiden künstlichen Kreuzbänder kombiniert und dadurch kann der operative Eingriff für den Ersatz eines Kniegelenkes durch eine Kniegelenk-Endoprothese wesentlich vereinfacht werden, da nunmehr der gesamte natürliche Tibiakopf des Schienbeines einschließlich des natürlichen Höckersteges mittels eines ebenen Schnittes reseziert wird. Sodann werden die Schalen mit den Gleitflächen und dem künstlichen Höckersteg mit den an ihm befestigten unteren Enden der künstlichen Kreuzbänder im Schienbein verankert.

Vorzugsweise geht man nach der Erfindung so vor, daß der Höckersteg auf der Unterseite seitlich der Mittellinie des Höckersteges je eine Einbohrung für das Einsetzen je eines mit dem einen Ende jeder Kreuzbänder verbundenen Zapfens aufweist, von denen ausgehend je eine Nut zu aufrechten Durchbrechungen des Höckersteges für die Durchführung der Kreuzbänder verläuft. Weitere Merkmale ergeben sich aus den nachstehenden Erläuterungen.

Die Erfindung wird nachstehend anhand der Zeichnung erläutert, in der ein Ausführungsbeispiel dargestellt ist. Es zeigen:

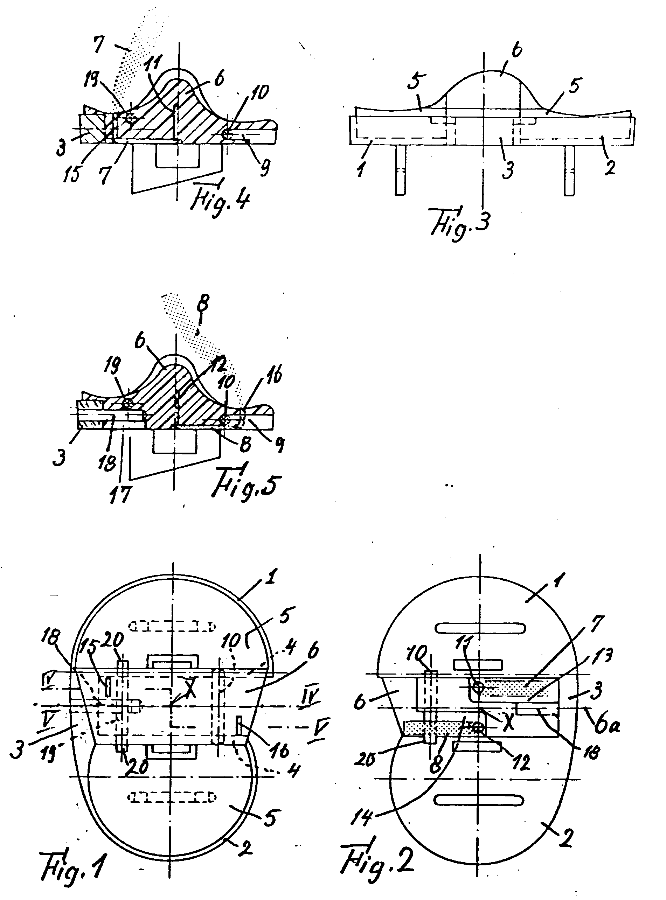

Figur 1      eine Aufsicht auf das Tibiateil entsprechend der Erfindung,

Figur 2      eine Unteransicht des Tibiateiles nach Figur 1,

Figur 3      eine Voransicht des Tibiateiles nach Figur 1,

Figur 4      einen Schnitt nach der Linie IV-X-IV der Figur 1 und

Figur 5      einen Schnitt nach der Linie V-X-V der Figur 1.

Das Tibiateil der Kniegelenk-Endoprothese besteht aus den beiden metallischen Schalen 1 und 2, die vorn bei 3 überbrückt verbunden sind und deren einander zugekehrte Seitenwandungen 4 parallel verlaufen. Die Schalen 1,2 nehmen die aus Kunststoff bestehenden Tibiaplateaus 5 mit Gleitflächen für die Kufen des Femur-Prothesenteiles auf. Die Schalen 1,2 sind in bekannter Weise im Schienbein zu verankern.

Der freie Raum zwischen den beiden Schalen 1,2 und der vorderen Überbrückung 3 ist von einem Höckersteg 6 aus einem Kunststoff, wie Polyäthylen, ausgefüllt, wobei der Höckersteg einem natürlichen Höckersteg nachgebildet ist. Die Fläche des Höckersteges 6 geht in die Oberflächen der beiden Plateaus 5 über.

Der Höckersteg 6 dient zur Befestigung der unteren Enden der beiden aus einem Kunststoff bestehenden Kreuzbänder 7 und 8, die die natürlichen, verschlissenen Kreuzbänder ersetzen.

Zu diesem Zweck ist der Höckersteg 6 zwischen den beiden Schalen 1,2 einzusetzen und zu fixieren. Der Höckersteg 6 wird unten durch Formschluß mit den Schalen 1,2 verbunden, indem er auf der hinteren Unterseite mit einem von hinten nach vorn gerichteten Schlitz 9 versehen ist, mit dem der Höckersteg in geneigter Lage über einen Querzapfen 10 zwischen die beiden Schalen 1,2 geschoben wird. Weiter ist der Höckersteg 6 auf der Unterseite beidseitig einer längsverlaufenden Mittellinie 6a mit zwei Einbohrungen 11 und 12 versehen, in die Zapfen einsteckbar sind, mit welchem die unteren Enden der beiden Kreuzbänder 7 und 8 fest verbunden sind. Die beiden Kreuzbänder 7,8 verlaufen von den Bohrungen 11,12 aus entgegengesetzt in Nuten 13,14 auf der Unterseite des Höckersteges 6 bis zu aufrechten Durchbrechungen 15 und 16 des Höckersteges und von dort nach oben. Die oberen Enden der beiden Kreuzbänder 7 und 8 werden mit dem nicht dargestellten Femurteil verbunden.

Der Höckersteg 6 mit den beiden Kreuzbändern 7,8 wird um den Querzapfen 10 aus der erwähnten geneigten Lage in den Raum zwischen die beiden Schalen 1,2 verschwenkt. Dabei übergreift ein vorderer unterer Ausschnitt 17 des Höckersteges 6 einen in der Brücke 3 befestigten Zapfen 18, so daß der Zapfen 18 durch die Wandungen des Ausschnittes festgeklemmt wird.

Weiter ist im Vorderteil des Höckersteges 6 ein quer verlaufender Zapfen 19 vorgesehen, dessen beide freie Enden beidseitig des Höckersteges 6 in obere Ausnehmungen 20 der beiden Tibiaplateaus 5 eingreifen und dadurch die Plateaus in den beiden Schalen 1,2 fixieren.

## Patentansprüche

1. Tibiateil einer Kniegelenkendoprothese, bei dem in die beiden vorn durch eine Brücke (3) zusammenhängenden im Schienbein zu verankernden, metallischen Schalen (1,2) die Tibiaplateaus (5) mit Gleitflächen für die Kufen des Femur-Endoprothesenteiles eingesetzt sind, dadurch gekennzeichnet, daß der Raum zwischen den beiden Schalen (1,2) von einem künstlichen Höckersteg (6) ausgefüllt ist, der hinten durch Formschluß mit den Schalen verbindbar ist und vorn durch eine Klemmverbindung an der Schalenbrücke (3) befestigt ist und der mit den Enden von künstlichen Kreuzbändern (7,8) verbindbar ist.

2. Tibiateil nach Anspruch 1, dadurch gekennzeichnet, daß der Formschluß aus einem Querzapfen (10) zwischen den beiden Schalen (1,2) und aus einem von hinten nach vorn gerichteten, über den Querzapfen (10) greifenden Schlitz (9) des Höckersteges (6) besteht.

3. Tibiateil nach einem der Ansprüche 1 und 2, dadurch gekennzeichnet, daß der vordere Klemmverschluß aus einem in der Schalenbrücke (3) verankerten, in den Freiraum zwischen den beiden Schalen (1,2) gerichteten Zapfen (18) besteht, der in einer unteren Ausnehmung (17) des Höckersteges festklemmbar eingreift.

4. Tibiateil nach einem der Ansprüche 1 bis 3, dadurch gekennzeichnet, daß der Höckersteg (6) auf der Unterseite seitlich der Mittellinie (6a) des Steges (6) je eine Einbohrung für das Einsetzen je eines mit dem einen Ende jeder Kreuzbänder (7,8) verbundenen Zapfens (11,12) aufweist, von denen ausgehend je eine Nut (13,14) zu den aufrechten Durchbrechungen (15,16) des Höckersteges (6) für die Durchführung der Kreuzbänder (7,8) verläuft.

5. Tibiateil nach einem der Ansprüche 1 bis 4, dadurch gekennzeichnet, daß im Vorderteil des Höckersteges (6) ein Querzapfen (19) vorgesehen ist, dessen beide freie Enden in obere Ausnehmungen (20) der beiden Tibiaplateaus (5) eingreifen.

## Claims

1. A part for the tibia in a kneejoint endoprosthesis, in which the tibia plateaus (5) having sliding faces for the skids on the femur part of the endoprosthesis are inserted in the two metal shells (1, 2) for anchoring in the shin-bone which are connected at the front by a bridge (3), characterized in that the space between the two shells (1, 2) is filled with a protuberant web (6) of plastics which may be connected to the shells at the rear with a close fit and is fastened to the shell bridge (3) at the front by a clamp connection and may be connected to the ends of crucial ligaments (7, 8) of plastics.

2. A part for the tibia as in Claim 1, characterized in that the close fit consists of a crosspin (10) between the two shells (1, 2) and a slot (9) in the protuberant web (6) directed from the rear towards the front and engaging over the crosspin (10).

3. A part for the tibia as in one of the Claims 1 and 2, characterized in that the clamp closure at the front consists of a pin (18) which is anchored in the shell bridge (3) and directed into the free space between the two shells (1, 2) and may be firmly clamped by engaging in a recess (17) at the bottom of the protuberant web.

4. A part for the tibia as in one of the Claims 1 to 3, characterized in that on the underside at each side of the centreline (6a) of the protuberant web (6) the web (6) exhibits a hole drilled in it for inserting a pin (11, 12) which is connected to one end of a respective crucial ligament (7, 8) and starting from which a groove (13, 14) runs to the vertical openings (15, 16) in the protuberant web (6) for leading through the crucial ligaments (7, 8).

5. A part for the tibia as in one of the Claims 1 to 4, characterized in that in the front part of the protuberant web (6) a crosspin (19) is provided, the two free ends of which engage in recesses (20) in the tops of the two tibia plateaus (5).

**Revendications**

1. Partie de tibia d'une endoprothèse pour articulation de genou dans laquelle les plateaux de tibia (5) présentant des surfaces de glissement pour les patins de la partie de fémur de l'endoprothèse sont mis en oeuvre dans les deux coques métalliques (1, 2) qui sont reliées à l'avant par un pont (3) et sont à ancrer dans l'os de la jambe, caractérisée en ce que l'espace compris entre les deux coques (1, 2) est rempli par une nervure en saillie (6) artificielle qui peut être reliée à l'arrière, par une liaison due à la forme, aux coques, est fixée à l'avant, par une liaison par pinçage, sur le pont (3), et peut être reliée aux extrémités de bandes en croix synthétiques (7, 8).

2. Partie de tibia suivant la revendication 1, caractérisée en ce que la liaison par la forme est constituée d'un tourillon transversal (10) entre les deux coques (1, 2) et d'une fente (9) de la nervure en saillie (6) qui se dirige de l'arrière vers l'avant et prend pardessus le tourillon transversal (10).

3. Partie de tibia suivant l'une des revendications 1 et 2, caractérisée en ce que la liaison avant par pinçage est constituée d'un tenon (18) qui est ancré dans le pont (3), est dirigé dans l'espace libre entre les deux coques (1, 2) et pénètre dans un évidement inférieur (17) de la nervure en saillie de manière à pouvoir y être pincé à bloc.

4. Partie de tibia suivant l'une des revendications 1 à 3, caractérisée en ce que la nervure en saillie (6) présente sur la face inférieure, latéralement à la ligne centrale (6a) de la nervure (6), à chaque fois une perforation pour l'introduction de chaque fois un tenon (11, 12) relié à une extrémité de chacune des bandes en croix (7, 8), perforations à partir de chacune desquelles une rainure (13, 14) s'étend vers les perforations verticales (15, 16) de la nervure en saillie (6) pour le passage des bandes en croix (7, 8).

5. Partie de tibia suivant l'une des revendications 1 à 4, caractérisée en ce que dans la partie avant de la nervure en saillie (6) est prévu un tourillon transversal (19) dont les deux extrémités libres pénètrent dans des évidements supérieurs (20) des deux plateaux de tibia (5).

Fig.4

Fig.3

Fig.5

Fig.1

Fig.2